(19) Europäisches Patentamt
European Patent Office
Office européen des brevets

(11) Veröffentlichungsnummer : **0 417 473 B1**

(12) # EUROPÄISCHE PATENTSCHRIFT

(45) Veröffentlichungstag der Patentschrift : **15.09.93 Patentblatt 93/37**

(51) Int. Cl.$^5$ : **A61K 37/64,** C07K 5/06, A61K 37/02

(21) Anmeldenummer : **90115230.6**

(22) Anmeldetag : **08.08.90**

(54) **Verfahren zur Behandlung der cardialen sowie der vasculären Hypertrophie und Hyperplasie.**

Die Akte enthält technische Angaben, die nach dem Eingang der Anmeldung eingereicht wurden und die nicht in dieser Patentschrift enthalten sind.

(30) Priorität : **11.08.89 DE 3926606**

(43) Veröffentlichungstag der Anmeldung : **20.03.91 Patentblatt 91/12**

(45) Bekanntmachung des Hinweises auf die Patenterteilung : **15.09.93 Patentblatt 93/37**

(84) Benannte Vertragsstaaten : **AT BE CH DE DK ES FR GB GR IT LI LU NL SE**

(56) Entgegenhaltungen :
**EP-A- 0 279 357**
**EP-A- 0 363 671**
**CHEMICAL ABSTRACTS Band 111, Nr. 3, 17. Juli 1989, Seite 40, Zusammenfassung Nr. 17453h, Columbus, Ohio, US; J.P. CLOZEL et al.: "Effects of chronic ACE inhibition on cardiac hypertrophy and coronary vascular reserve in spontaneously hypertensive rats with developed hypertension"**
**CHEMICAL ABSTRACTS Band 109, Nr. 19, 7. November 1988, Seite 45, Zusammenfassung Nr. 163232k, Columbus, Ohio, US; G.P. HODSMAN et al.: "Cardiac hypertrophy and salt status in chronic myocardial infarction in the rat: effects of enalapril versus salt restriction"**
**CHEMICAL ABSTRACTS Band 110, Nr. 19, 8. Mai 1989, Seite 49, Zusammenfassung Nr. 165828d, Columbus, Ohio, US; D. FERNANDEZ et al.: "Modulation of left ventricular hypertrophy by dietary salt and inhibition of angiotensin converting enzyme"**

(56) Entgegenhaltungen :
**CHEMICAL ABSTRACTS Band 113, Nr. 7, 13. August 1990, Seiten 37,38, Zusammenfassung Nr. 52297y, Columbus, Ohio, US; J.B. MICHEL: "Relationship between decrease in afterload and beneficial effects of ACE inhibitors in experimental cardiac hypertrophy and congestive heart failure"**

(73) Patentinhaber : **HOECHST AKTIENGESELLSCHAFT**
**D-65926 Frankfurt (DE)**

(72) Erfinder : **Linz, Wolfgang, Dr.**
**Huxelrebenweg 54**
**D-6500 Mainz (DE)**
Erfinder : **Schölkens, Bernward, Prof. Dr.**
**Hölderlinstrasse 62**
**D-6233 Kelkheim (Taunus) (DE)**
Erfinder : **Scholz, Wolfgang, Dr.**
**Unterortstrasse 30**
**D-6236 Eschborn (DE)**
Erfinder : **Wiemer, Gabriele, Dr.**
**Ernst-Moritz-Arndt-Strasse 21**
**D-6242 Kronberg/Taunus (DE)**
Erfinder : **Urbach, Hansjörg, Dr.**
**Le Lavandoustrasse 41**
**D-6242 Kronberg/Taunus (DE)**
Erfinder : **Henning, Rainer, Dr.**
**Lorsbacher Strasse 4g**
**D-6234 Hattersheim (DE)**
Erfinder : **Teetz, Volker, Dr.**
**An der Tann 20**
**D-6238 Hofheim am Taunus (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft ein Verfahren zur Behandlung der cardialen sowie der vasculären Hypertrophie und/oder Hyperplasie durch perorale oder parenterale Anwendung von Verbindungen, die das Angiotensin-Converting-Enzym hemmen.

In EP-A 363 671 werden zur Behandlung des Bluthochdrucks bekannte ACE-Inhibitoren beschrieben sowie deren Verwendung zur Therapie von Gefäßverletzungen. Aus J. Hypertens. (1989, 7(4), 267-275) sowie aus J. Cardiovasc. Pharmacol. (1988, 12(4), 467-472) sowie aus J. Hypertens. (1988, 6(4), 145-147) ist bekannt, daß spezielle ACE-Inhibitoren zur Behandlung des Bluthochdrucks wie auch der cardialen Hypertrophie eingesetzt werden können. Die vorliegende Erfindung betrifft den Einsatz spezieller ACE-Inhibitoren zur Behandlung der Hypertrophie und Hyperplasie unter Anwendung niedriger, sub-antihypertensiv wirksamer Dosen .

Dabei kommen besonders Verbindungen der Formel I in Betracht,

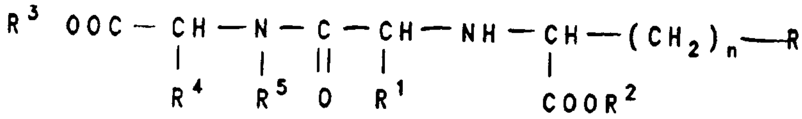

(I)

in welcher

n= 1 oder 2 ist,

R= Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen, das wie oben bei Aryl beschrieben substituiert sein kann,
Amino-$(C_1-C_4)$-alkyl,
$(C_1-C_4)$-Alkanoylamino-$(C_1-C_4)$-alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben substituiert sein kann, $(C_6-C_{12})$-Aryl-$(C_1-C_4)$-alkylthio,
das im Arylteil wie oben bei Aryl beschrieben substituiert sein kann. Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryl beschrieben substituiert sein kann oder $(C_6-C_{12})$-Aryl-$(C_1-C_4)$-alkoxy,
das im Arylteil wie oben bei Aryl beschrieben substituiert sein kann,

$R^1$ Wasserstoff,

Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6-12 C-Atomen,
das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)-alkyl, die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder
die gegebenenfalls geschützte Seitenkette einer natürlich vorkommenden α-Aminosäure $R^1$-$CH(NH_2)$-COOH
bedeuten,

$R^2$ und $R^3$ gleich oder verschieden sind und Wasserstoff,
Alkyl mit 1-6 C-Atomen,
Alkenyl mit 2-6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy-($C_1$-$C_4$)-alkyl,
Aryl mit 6-12 C-Atomen,
($C_6$-$C_{12}$)-Aryl-($C_1$-$C_4$)-alkyl,
($C_3$-$C_9$)-Cycloalkyl oder
($C_3$-$C_9$)-Cycloalkyl-($C_1$-$C_4$)-alkyl
bedeuten und worin

$R^4$ und $R^5$ zusammen mit den sie tragenden Atomen für ein gegebenenfalls substituiertes System aus der Reihe Tetrahydroisochinolin, Decahydroisochinolin, Octahydroindol, Octahydrocyclopenta(b) pyrrol, 2-Azaspiro(4,4)nonan, Spiro[(bicyclo(2.2.1)heptan)-2.3-pyrrolidin], Spiro[(bicyclo-(2,2,2) octan)-2.3-pyrrolidin], 2-Azatricyclo 4.3.0.1$^{6.9}$)decan, Decahydrocyclohepta(b)pyrrol, Octahydroisoindol, Octahydrocyclopenta(c)pyrrol, 2.3.3a.4.5.7a-Hexahydroindol und 2-Azabicyclo[3. 1.0]hexan stehen.

Bei den Verbindungen, die mehrere chirale Atome besitzen kommen alle möglichen Diastereomere als Racemate oder Enantiomere, oder Gemische verschiedener Diastereomere in Betracht.

Die in Betracht kommenden cyclischen Aminosäureester weisen die folgenden Strukturformeln auf:

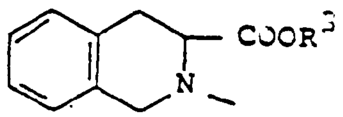

A

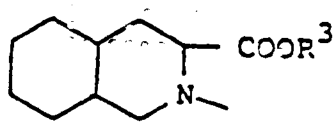

B

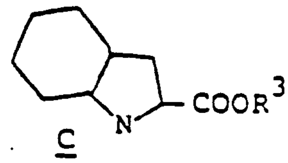

C

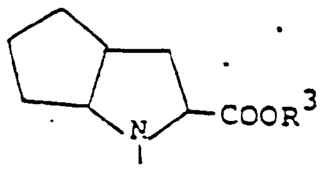

D

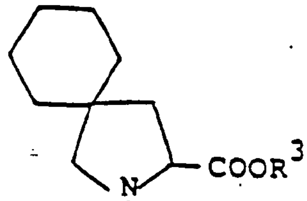

E

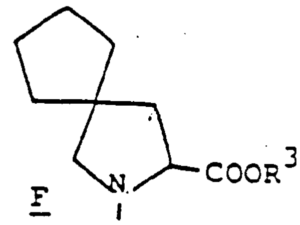

F

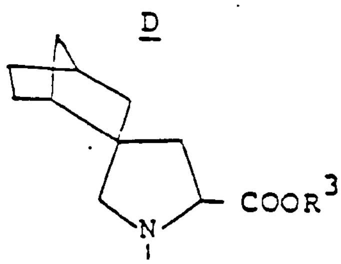

G

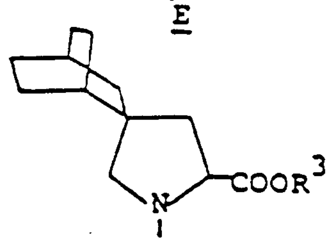

H

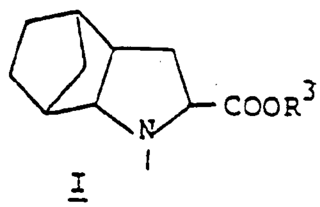

I

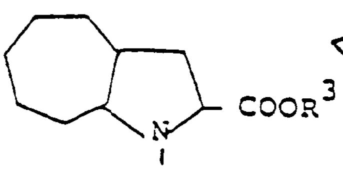

J

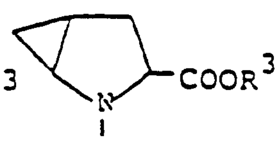

K

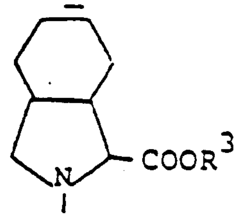

L

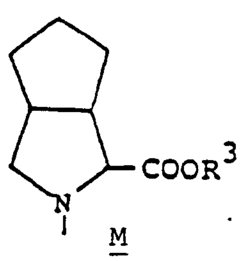

M

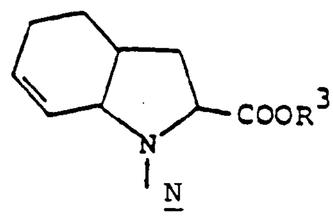

N

Besonders bevorzugt ist eine Ausführungsform, die dadurch gekennzeichnet ist, daß Verbindungen der Formel I angewendet werden, in der

n = 1 oder 2 ist,

R = $(C_1-C_6)$-Alkyl, $(C_2-C_6)$-Alkenyl, $(C_3-C_9)$-Cycloalkyl Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono, di- oder trisubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,

$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl,$(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$-alkyl, die beide wie vorstehend definiert im Arylrest substituiert sein

können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, $(C_1-C_3)$-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, $(C_1-C_6)$-Alkyl, $(C_2-C_6)$-Alkenyl oder $(C_6-C_{12})$-Aryl-$(C_1-C_4)$-alkyl, insbesondere aber Wasserstoff, $(C_1-C_4)$-Alkyl oder Benzyl bedeuten und

$R^4$ und $R^5$ die oben angegebene Bedeutung haben.

Insbesondere bevorzugt ist die Anwendung von Verbindungen der Formel I, in welcher n= 2 ist, R= Phenyl, $R^1$= Methyl, $R^2$ und $R^3$ gleiche oder verschiedene $(C_1-C_6)$-Alkylreste oder $(C_7-C_{10})$-Aralkylreste wie Benzyl oder Nitrobenzyl bedeuten und

$R^4$ und $R^5$ zusammen für einen Rest der Formel steht,

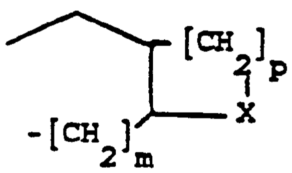

worin m= 0 oder 1, p= 0, 1 oder 2 und X= $-CH_2-$, $-CH_2-CH_2-$ oder -CH=CH- bedeuten, wobei ein mit X gebildeter 6-Ring auch ein Benzolring sein kann.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl,Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen in der Peptidchemie üblichen Grupppen bevorzugt (vgl. Houben-Weyl, Bd. XV/1 und XV/2). Im Falle, daß $R^1$ die geschützte Lysin Seitenkette bedeutet, werden die bekannten Amino-Schutzgruppen, insbesondere aber Z, Boc oder $(C_1-C_6)$-Alkanoyl bevorzugt. Als O-Schutzgruppen für Tyrosin kommen bevorzugt $(C_1-C_6)$-Alkyl, insbesondere Methyl oder Ethyl in Frage.

Besonders vorteilhaft können die folgenden Verbindungen nach der erfindungsgemäßen Methode angewendet werden:

N-(1-S-Carbethoxy-3-phenyl-propyl)-S-alanyl-S-1,2,3,4-tetrahydroisochinolin-3-carbonsäure
N-(1-S-Carbethoxy-3-cyclohexyl-propyl)-S-alanyl-S-1,2,3,4-tetrahydroisochinolin-3-carbonsäure
N-(1-S-Carbethoxy-3-phenyl-propyl)-S-lysyl-S-1,2,3,4-tetrahydroisochinolin-3-carbonsäure
N-(1-S-Carbethoxy-3-phenyl-propyl)-O-ethyl-S-tyrosyl-S-1,2,3,4-tetrahydroisochinolin-3-carbonsäure
N-(1-S-Carbethoxy-3-phenyl-propyl)-S-alanyl-3S-decahydroisochinolin-3-carbonsäure
N-(1-S-Carbethoxy-3-phenyl-propyl)-S-alanyl-(2S,3aS,7aS)-octahydroindol-2-carbonsäure
N-(1-S-Carbethoxy-3-cyclohexyl-propyl)-S-alanyl-(2S,3aS, 7aS)-octahydroindol-2-carbonsäure
N-(1-S-Carbethoxy-3-phenyl-propyl)-S-lysyl-(2S,3aS,7aS)-octahydroindol-2-carbonsäure
N-(1-S-Carbethoxy-3-cyclohexyl-propyl)-S-lysyl-(2S,3aS, 7aS)-octahydroindol-2-carbonsäure
N-(1-S-Carbethoxy-3-cyclohexyl-propyl)-S-lysyl-(2S,3aS, 7aS)-octahydroindol-2-carbonsäure
N-(1-S-Carbethoxy-3-phenyl-propyl)-O-methyl-S-tyrosyl-(2S, 3aS,7aS)-octahydroindol-2-carbonsäure
N-(1-S-Carbethoxy-3-phenyl-propyl)-O-ethyl-S-tyrosyl-(2S,3aS,7aS)-octahydroindol-2-carbonsäure
N-(1-S-Carbethoxy-3-(3,4-dimethylphenyl-propyl)-S-alanyl-(2S,3aS,7aS)-octahydroindol-2-carbonsäure
N-[1-S-Carbethoxy-3-(4-fluorphenyl)-propyl]-S-alanyl-(2S,3aS,7aS)-octahydroindol-2-carbonsäure,
N-[1-S-Carbethoxy-3-(4-methoxyphenyl)-propyl]-S-alanyl-(2S,3aS,7aS)-octahydroindol-2-carbonsäure
N-[1-S-Carbethoxy-3-(3,4-dimethoxyphenyl)-propyl]-S-alanyl-(2S,3aS,7aS)-octahydroindol-2-carbonsäure

N-(1-S-Carbethoxy-3-cyclopentylpropyl)-S-alanyl-(2S,3aS, 7aS)-octahydroindol-2-carbonsäure
N-(1-S-Carbethoxy-3-phenyl-propyl)-S-alanyl-(2S,3aR,7aS)-octahydroindol-2-carbonsäure
N-(1-S-Carbethoxy-3-cyclohexyl-propyl)-S-alanyl-(2S,3aR,7aS)-octahydroindol-carbonsäure
N-(1-S-Carbethoxy-3-phenyl-propyl)-S-lysyl-(2S,3aR,7aS)-octahydroindol-2-carbonsäure
N-(1-S-Carbethoxy-3-cyclohexyl-propyl)-S-lysyl-(2S,3aR, 7aS)-octahydroindol-2-carbonsäure
N-(1-S-Carbethoxy-3-phenyl-propyl)-O-ethyl-S-tyrosyl-(2S,3aS,7aR)-octahydroindol-2-carbonsäure
N-(1-S-Carbethoxy-3-phenyl-propyl)-S-alanyl-(2S,3aR,7aR)-octahydroindol-2-carbonsäure
N-(1-S-Carbethoxy-3-phenyl-propyl)-S-lysyl-(2S,3aR,7aS)octahydroindol-2-carbonsäure
N-(1-S-Carbethoxy-3-cyclohexyl-propyl)-S-alanyl-(2S,3aR, 7aR)-octahydroindol-2-carbonsäure
N-(1-S-Carbethoxy-3-cyclohexyl-propyl)-O-ethyl-S-tyrosyl-(2S,3aR,7aR)-octahydroindol-2-carbonsäure
N-(1-S-Carbethoxy-3-phenyl-propyl)-S-alanyl-(2S,3aS,7aR)-octahydroindol-2-carbonsäure
N-(1-S-Carbethoxy-3-phenyl-propyl)-O-ethyl-S-tyrosyl-(2S,3aS,7aS)-octahydroindol-2-carbonsäure
N-(1-S-Carbethoxy-3,4-dimethylphenyl-propyl)-S-alanyl-(2S,3aS,7aS)-octahydroindol-2-carbonsäure
N-[1-S-Carbethoxy-3-(4-fluorphenyl)-propyl]-S-alanyl-(2S,3aS,7aS)-octahydroindol-2-carbonsäure
N-[1-S-Carbethoxy-3-(4-methoxyphenyl)-propyl]-S-alanyl-(2S,3aS,7aS)-octahydroindol-2-carbonsäure
N-[1-S-Carbethoxy-3-(3,4-dimethoxyphenyl)-propyl]-S-alanyl-(2S,3aS,7aS)-octahydroindol-2-carbonsäure
N-(1-S-Carbethoxy-3-cyclopentylpropyl)-S-alanyl-(2S,3aS, 7aS)-octahydroindol-2-carbonsäure
N-(1-S-Carbethoxy-3-phenyl-propyl)-S-alanyl-cis-endo-2-azabicyclo[3.3.0]octan-3-S-carbonsäure
N-(1-S-Carbethoxy-3-phenyl-propyl)-S-lysyl-cis-endo-2-azabicyclo[3.3.0]octan-3-S-carbonsäure
N-(1-S-Carbethoxy-3-cyclohexyl-propyl)-S-alanyl-cis-endo-2-azabicyclo[3.3.0]octan-3-S-carbonsäure
N-(1-S-Carboxy-3-cyclohexyl-propyl)-S-alanyl-cis-endo-2-azabicyclo[3.3.0]octan-3-S-carbonsäure
N-(1-S-Carbethoxy-butyl)-S-alanyl-cis-endo-2-azabicyclo[3.3.0]octan-3-S-carbonsäure
N-(1-S-Carbethoxy-3-(3,4-dimethoxyphenylpropyl)-S-alanyl-cis-endo-2-azabicyclo[3.3.0]octan-3-S-carbonsäure
N-(1-S-Carbethoxy-3-cyclopentyl-propyl)-S-alanyl-cis-endo-azabicyclo-[3.3.0]octan-3-S-carbonsäure
N-(1-S-Carbethoxy-3-phenyl-propyl)-O-methyl-S-tyrosyl-cis-endo-2-azabicyclo[3.3.0]octan-3-S-carbonsäure
N-(1-S-Carbethoxy-3-phenyl-propyl)-O-ethyl-S-tyrosyl-cis-endo-2-azabicyclo[3.3.0]octan-3-S-carbonsäure
N-(1-S-Carbethoxy-3-(4-fluorphenyl-propyl)-S-alanyl-cis-endo-azabicyclo[3.3.0]octan-3-S-carbonsäure
N-(1-S-Carbethoxy-3-(4-methoxyphenyl-propyl)-S-alanyl-cis-endo-2-azabicyclo[3.3.0]octan-3-S-carbonsäure
N-(1-S-Carbethoxy-3-phenyl-propyl)-S-lysyl-(2S,3aR,6aS)-octahydrocyclopenta[b]pyrrol-2-carbonsäure
N-(1-S-Carbethoxy-3-cyclohexylpropyl)-lysyl-(2S,3a,6aS)-octahydrocyclopenta[b]pyrrol-2-carbonsäure
N-(1-S-Carbethoxy-3-phenyl-propyl)-O-ethyl-S-tyrosyl-(2S,3aR,6aS)-octahydrocyclopenta[b]pyrrol-2-carbonsäure
N-(1-S-Carbethoxy-3-phenyl-propyl)-S-alanyl-2-(2S,3aR,6aS)-octahydrocyclopenta[b]pyrrol-2-carbonsäure
N-(1-S-Carbethoxy-3-phenyl-propyl)-S-alanyl-2-azaspiro-[4,5]decan-3-S-carbonsäure
N-(1-S-Carbethoxy-3-phenyl-propyl)-O-ethyl-2-tyrosyl-azaspiro-[4,5]decan-3-S-carbonsäure
N-(1-S-Carbethoxy-3-phenyl-propyl)-S-lysyl-2-azaspiro-[4,5]decan-3-S-carbonsäure
N-(1-S-Carbethoxy-3-cyclohexylpropyl)-S-alanyl-2-azaspiro [4,5]decan-3-S-carbonsäure
N-(1-S-Carbethoxy-3-cyclohexylpropyl)-S-lysyl-2-azaspiro-[4,5]decan-3-S-carbonsäure
N-(1-S-Carbethoxy-3-phenyl-propyl)-S-lysyl-2-azaspiro-[4,4]nonan-3-S-carbonsäure
N-(1-S-Carbethoxy-3-phenyl-propyl)-O-ethyl-S-tyrosyl-2-azaspiro[4,4]nonan-3-S-carbonsäure
N-(1-S-Carbethoxy-3-phenyl-propyl)-S-lysyl-2-azaspiro-[4,4]nonan-3-S-carbonsäure
N-(1-S-Carbethoxy-3-cyclohexyl-propyl)-S-alanyl-2-azaspiro[4,4]nonan-3-S-carbonsäure
N-(1-S-Carbethoxy-3-cyclopentyl-propyl)-S-alanyl-2-azaspiro[4,4]nonan-3-S-carbonsäure
N-(1-S-Carbethoxy-3-cyclopentyl-propyl)-S-lysyl-2-azaspiro[4,4]nonan-3-S-carbonsäure
N-(1-S-Carbethoxy-3-phenyl-propyl)-S-alanyl-spiro[bicyclo[2.2.1]heptan-2,3'-pyrrolidin]-5'-S-carbonsäure
N-(1-S-Carbethoxy-3-phenyl-propyl)-O-ethyl-S-tyrosyl-spiro[bicyclo[2.2.1]heptan-2,3'-pyrrolidin]-5'-S-carbonsäure
N-(1-S-Carbethoxy-3-phenyl-propyl)-S-lysyl-spiro[bicyclo[2.2.1]heptan-2,3'-pyrrolidin]-5'-S-carbonsäure
N-(1-S-Carbethoxy-3-cyclohexyl-propyl)-S-alanyl-spiro[bicyclo[2.2.1]heptan-2,3'-pyrrolidin]5'-S-carbonsäure
N-(1-S-Carbethoxy-3-cyclohexyl-propyl)-S-lysyl-spiro-[bicyclo[2.2.1]heptan-2,3'-pyrrolidin]-5'-S-carbonsäure
N-(1-S-Carbethoxy-3-phenyl-propyl)-S-alanyl-spiro[bicyclo[2.2.2]octan-2,3'-pyrrolidin]-5'-S-carbonsäure
N-(1-S-Carbethoxy-3-phenyl-propyl)-O-ethyl-tyrosyl-spiro[bicyclo[2.2.2]octan-2,3'-pyrrolidin]-5'-S-carbonsäure
N-(1-S-Carbethoxy-3-phenyl-propyl)-S-lysyl-spiro[bicyclo[2.2.2]octan-2,3'-pyrrolidin]-5'-S-carbonsäure
N-(1-S-Carbethoxy-3-cyclohexyl-propyl)-S-alanyl-spiro[bicyclo[2.2.2]octan-2,3'-pyrrolidin]-5'-S-carbonsäure
N-(1-S-Carbethoxy-3-phenyl-propyl)-S-alanyl-2-azatricyclo[4,3,0,$1^{6,9}$]decan-3-S-carbonsäure
N-(1-S-Carbethoxy-3-phenyl-propyl)-O-ethyl-S-tyrosyl-2-azatricyclo[4,3,0,$1^{6,9}$]decan-3-S-carbonsäure
N-(1-S-Carbethoxy-3-phenyl-propyl)-S-lysyl-2-azatricyclo[4,3,0,$1^{6,9}$]decan-3-S-carbonsäure
N-(1-S-Carbethoxy-3-cyclohexyl-propyl)-S-alanyl-2-azatricyclo[4,3,0,$1^{6,9}$]decan-3-S-carbonsäure
N-(1-S-Carbethoxy-3-cyclohexyl-propyl)-S-lysyl-2-azatricyclo[4,3,0,$1^{6,9}$]decan-3-S-carbonsäure

N-(1-S-Carbethoxy-3-phenyl-propyl)-S-alanyl-decahydrocyclohepta[b]pyrrol-2-S-carbonsäure
N-(1-S-Carbethoxy-3-phenyl-propyl)-O-ethyl-S-tyrosyl-decahydrocyclophepta[b]pyrrol-2-S-carbonsäure
N-(1-S-Carbethoxy-3-phenyl-propyl)-S-lysyl-decahydrocyclohepta[b]pyrrol-2-S-carbonsäure
N-(1-S-Carbethoxy-3-cyclohexyl-propyl)-S-alanyl-decahydrocyclohepta[b]pyrrol-2-S-carbonsäure
N-(1-S-Carbethoxy-3-cyclohexyl-propyl)-S-lysyl-decahydrocyclohepta[b]pyrrol-2-S-carbonsäure
N-(1-S-Carbethoxy-3-phenyl-propyl)-S-alanyl-trans-octahydroisoindol-1-S-carbonsäure
N-(1-S-Carbethoxy-3-phenyl-propyl)-S-alanyl-cis-octahydroisoindol-1-S-carbonsäure
N-(1-S-Carbethoxy-3-cyclohexyl-propyl)-S-alanyl-transoctahydroisoindol-1-S-carbonsäure
N-(1-S-Carbethoxy-3-cyclohexyl-propyl)-S-alanyl-cis-octahydroisoindol-1-S-carbonsäure
N-(1-S-Carbethoxy-3-phenyl-propyl)-S-alanyl-cis-octahydrocyclopenta[c]pyrrol-1-S-carbonsäure
N-(1-S-Carbethoxy-3-cyclohexyl-propyl)-S-alanyl-cis-octahydrocyclopenta[c]pyrrol-1-S-carbonsäure-benzylester
N-(1-S-Carbethoxy-3-cyclohexyl-propyl)-S-lysyl-cis-octahydrocyclopenta[c]pyrrol-1-S-carbonsäure
N-(1-S-Carbethoxy-3-phenyl-propyl)-S-alanyl-2,3,3a,4,5,7a-hexahydroindol-cis-endo-2-S-carbonsäure
N-(1-S-Carbethoxy-3-phenyl-propyl)-S-lysyl-2,3,3a,4,5,7a-hexahydroindol-cis-endo-2-S-carbonsäure
N-(1-S-Carbethoxy-3-cyclohexyl-propyl)-S-lysyl-2-azabicyclo[3.1.0]hexan-3-S-carbonsäure
N-(1-S-Carboxy-3-phenyl-propyl)-S-lysyl-2-azabicyclo[3.1.0]hexan-cis-endo-3-S-carbonsäure
N-(1-S-Carbethoxy-3-cyclopentylpropyl)-S-alanyl-2-azabicylco[3.1.0]hexan-3-carbonsäure
N-(1-S-Carbethoxy-3-phenyl-propyl)-S-alanyl-cis-endo-2-azabicyclo[3.1.0]hexan-3-S-carbonsäure
N-(1-S-Carbethoxy-3-cyclohexyl-propyl)-S-alanyl-cis-endo-2-azabicyclo[3.1.0]hexan-3-S-carbonsäure

Diese Verbindungen lassen sich z.B. nach dem in er deutschen Patentanmeldung DE-A-33 33 455.2 beschriebenen Verfahren herstellen, in dem die in der Anmeldung beschriebenen tert. Butyl- oder Benzylreste in bekannter Weise durch saure oder alkalische Hydrolyse oder durch Edelmetall-katalysierte Hydrogenolyse in die Monocarbonsäurederivate überführt werden. Die $N^{\epsilon}$-Benzyloxycarbonylschutzgruppe der Lysinderivate wird durch Edelmetallkatalysierte Hydrogenolyse entfernt. Die oben aufgeführten Verbindungen lassen sich leicht mit physiologisch verträglichen Säuren oder Basen (im Falle von Mono- oder Dicarbonsäuren) in die entsprechenden Salze (z.B. Hydrochloride, Maleinate, Fumarate, etc.) überführen und als Salze die erfindungsgemäße Verwendung finden.

Die Verbindungen der Formel I sind Hemmstoffe des Angiotensin-converting-Enzymes (ACE) beziehungsweise Zwischenprodukte bei der Herstellung von solchen Hemmstoffen und können auch zur Bekämpfung des Bluthochdrucks verschiedener Genese eingesetzt werden. Die Verbindungen der Formel I sind z.B. bekannt aus US-Patent US-A-4 129 571, US Patent US-A-4 374 829, EP-A-79522, EP-A-79022, EP-A-49658, EP-A-51301, US Patent US-A-4 454 292, US-Patent US-A-4 374 847, EP-A-72352, US-Patent US-A-4 350 704, EP-A-50800, EP-A-46953, US-Patent US-A-4 344 949, EP-A-84164, US-Patent US-A-4 470 972, EP-A-65301 und EP-A-52991.

Vorteilhaft sind auch oral wirksame ACE-Inhibitoren, wie z.B. Ramipril, Enalapril, Captopril, Lisinopril, Perindopril, Cilazapril, RHC 3659, CGS 13945, CGS 13928C, CGS 14824A, CI-906, SCH 31846, Zofenopril, Fosenopril, Alacepril und andere. Oral wirksame ACE-Inhibitoren sind beispielsweise in Brunner et al., J. Cardiovasc. Pharmacol 7 (Suppl. I) [1985] S2-S11 beschrieben.

Bevorzugt sind die aus der EP-A-79022 bekannten ACE-Inhibitoren der Formel III

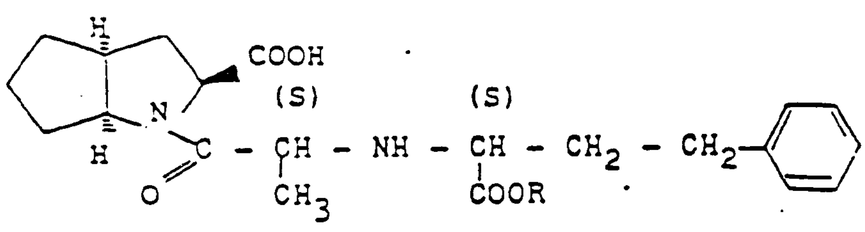

III)

in welcher
R Wasserstoff, Methyl, Ethyl oder Benzyl bedeutet,
insbesondere die Verbindung der Formel III, worin R=Ethyl bedeutet (Ramipril).

Weiterhin bevorzugt sind die aus der EP-A-84164 bekannten ACE-Inhibitoren der Formel IV

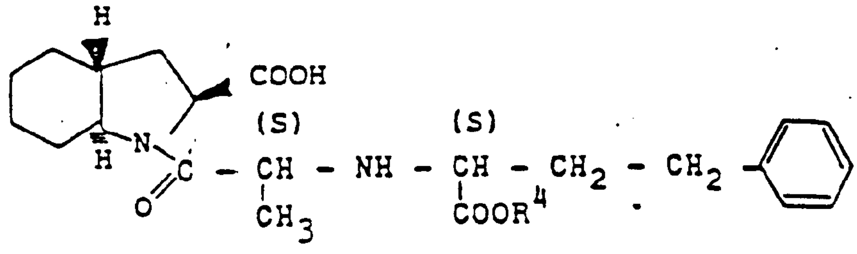

(IV)

in welcher
$R^4$ Wasserstoff, $(C_1-C_4)$-Alkyl oder Benzyl bedeutet, insbesondere die Verbindung der Formel IV, worin $R^4$ =Ethyl bedeutet.

In Ausübung der erfindungsgemäßen Methode können die oben beschriebenen Angiotensin-Converting-Enzyme-Inhibitoren an Säugern wie Affen, Hunden, Katzen, Ratten, Menschen etc. angewendet werden. Die für die erfindungsgemäße Verwendung geeigneten Verbindungen werden zweckmäßig in üblicher Weise in pharmazeutische Präparate eingearbeitet. Sie können in die üblichen Verabreichungsformen, wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können verträgliche, organische bzw. anorganische Begleitstoffe, beispielsweise Granulierstoffe, Klebe- und Bindemittel, Gleitmittel, Suspendiermittel, Lösungsmittel, antibakterielle Mittel, Netzmittel und Konservierungsmittel enthalten. Orale und parenterale Anwendungsformen werden bevorzugt. Die Verbindungen der Formel I können in Dosierungen von 0,001 mg/kg - 20 mg/kg, insbesondere 0,005 mg/kg - 1 mg/kg ein- bis dreimal täglich verabreicht werden.

In der Entwicklung der cardialen Hypertrophie als Folgeerscheinung einer Hypertonie sowie bei der Hypertrophie und Hyperplasie der glatten Gefäßmuskulatur, wie sie bei Hypertonie sowie bei der Ausbildung atherosklerotischer Plaqres beobachtet werden, spielen Wachstumsfaktoren, die zu einer Profiferation und zu einem Anschwellen der Zellen führen, eine entscheidende Rolle.

Aus der Literatur ist bekannt, daß Angiotensin II einen solchen Wachstumsfaktor darstellt. Behandlung von Muskelzellen mit Angiotensin 11 führt zur Stimulation von Phospholipase C (J. Biol. Chem. 260, 8901 (1986)), zur Mobilisierung von intracellulären Calcium (Hypertension 7, 447 (1988)), zur Aktivierung des $Na^+/M^+$-Austausches (J. Biol. Chem 262, 5057 (1987) sowie zur Aktivierung von Proteinsynthese und Induktion der messenger-RNA für das c-fos-protooncogen (J. Biol. Chem 264, 526 (1989)). Darüber hinaus potenziert Angiotensin II die proliberative Wirkung anderer Wachstumsfaktoren wie PDGF und EGF (Am. J. Physiol (1987), F 299). Diese beschriebenen Wirkungen des Angiotensin II werden hauptsächlich von lokalsynthetisiertem Peptid hervorgerufen. Verbindungen, die die Bildung von Angiotensin II lokal zu verhindern vermögen, sollten daher eine Wirkung auf die Hypertrophie und Hyperplasie des glatten Gefäßmuskels sowie des Herzmuskels aufweisen. Wir haben nun überraschend gefunden, daß Hemmer des Angiotensin-Converting-Enzymes bereits in Dosen, wo sie noch keine antihypertensive Wirkung zeigen, die beschriebenen trophischen Effekte des Angiotensin II aufzuheben vermögen.

Als Beispiele sollen im folgenden jeweils die Ergebnisse mit N-(1-S-Carbethoxy-3-phenyl-propyl)-S-alanyl-cis-endo-2-azabicyclo[3.3.0]octan-3-S-carbonsäure (Formel II) dienen.

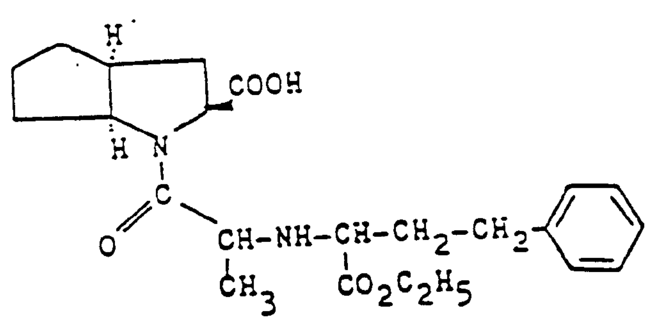

II

Versuchsbeschreibung

Bei wachen Ratten wurde durch Einengung der abdominalen Aorta eine Herzhypertrophie erzeugt. Nachdem diese voll etabliert war, erhielten Gruppen der Tiere für 3 Wochen 1 mg/kg/d (antihypertensive Dosis) bzw. 10 µg/kg/d (nicht antihypertensive Dosis) der Verbindung der Formel II durch perorale Gabe. Kontrollgruppen

ohne Substanzgabe und scheinoperierte Tiere wurden mitgeführt. Nach Ende der 3 Woche wurden die Tiere getötet und das Herzgewicht, die Wanddicke im linken Ventrikel und der myocardiale Proteingehalt bestimmt. In beiden behandelten Gruppen waren die erhobenen Werte signifikant gesenkt und nicht von den scheinoperierten Kontrollen zu unterscheiden.

Die folgenden Beispiele geben die Anwendungsformen zur Behandlung der cardialen sowie der vasculären Hypertrophie und Hyperplasie nach der erfindungsgemäßen Methode an. Die Verbindungen der Formel I können analog den Beispielen in die entsprechenden Anwendungsformen gebracht werden.

Beispiel 1

Herstellung des erfindungsgemäß verwendeten Mittels zur oralen Anwendung in der Behandlung der cardialen sowie der vasculären Hypertrophie und Hyperplasie.

1000 Tabletten, die je 10 mg 1-N-(1-S-Carbethoxy-3-phenyl-propyl)-S-alanyl-1S,3S,5S-2-azabicyclo[3.3.0] octan-3-carbonsäure enthalten, werden mit den folgenden Hilfsmitteln hergestellt:

| | |
|---|---|
| N-(1-S-Carbethoxy-3-phenyl-propyl)-S-alanyl-1S,3S,5S-2-azabicyclo[3.3.0]octan-3-carbonsäure | 10 g |
| Maisstärke | 140 g |
| Gelatine | 7,5 g |
| Mikrokristalline Cellulose | 2,5 g |
| Magnesiumstearat | 2,5 g |

N-(1-S-Carbethoxy-3-phenyl-propyl)-S-alanyl-1S,3S,5S-2-azabicyclo[3.3.0]octan-3-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg des ACE-Hemmers enthält.

Diese Tabletten können zur Behandlung der cardialen sowie der vasculären Hypertrophie und Hyperplasie verwendet werden.

Beispiel 2

Analog Beispiel 1 werden 1000 Tabletten hergestellt, die je 1 mg N-(1-S-Carbethoxy-3-phenyl-propyl)-S-alanyl-1S,3S,5S-2-azabicyclo[3.3.0]octan-3-carbonsäure enthalten, indem man von dieser Verbindung 1 g in den in Beispiel 1 beschriebenen Ansatz verwendet.

Beispiel 3

Analog Beispiel 1 werden 1000 Tabletten hergestellt, die je 10 mg N-(1-S-Carbethoxy-3-phenyl-propyl)-S-alanyl-(2S,3aR,7aS)-octahydroindol-2-carbonsäure-hydrochlorid enthalten.

Beispiel 4

Analog Beispiel 2 werden 1000 Tabletten hergestellt, die je 1 mg N-(1-S-Carbethoxy-3-phenyl-propyl)-S-alanyl-(2S,3aR,7aS)-octahydroindol-2-carbonsäure-hydrochlorid enthalten.

Beispiel 5

Gelatine-Kapseln, die je 10 mg N-(1-S-Carbethoxy-3-phenylpropyl)-S-alanyl-1S,3S,5S-2-azabicyclo[3.3.0]octan-3-carbonsäure enthalten, werden mit der folgenden Mischung gefüllt:

| | |
|---|---|
| N-(1-S-Carbethoxy-3-phenyl-propyl)-S-alanyl-1S,3S,5S-2-azabicyclo[3.3.0]octan-3-carbonsäure | 10 mg |
| Magnesiumstearat | 1 mg |
| Lactose | 214 mg |

Diese Kapseln können zur Behandlung der cardialen sowie der vasculären Hypertrophie und Hyperplasie verwendet werden.

Beispiel 6

Analog werden unter Verwendung von 10 mg Wirkstoff Gelatine-Kapseln hergestellt, die je 1 mg N-(1-S-Carbethoxy-3-phenyl-propyl)-S-alanyl-1S,3S,5S-2-azabicyclo[3.3.0]octan-3-carbonsäure enthalten.

Beispiel 7

Die Herstellung einer Injektionslösung zur Behandlung der cardialen sowie der vasculären Hypertrophie und Hyperplasie wird im folgenden beschrieben:

| | |
|---|---|
| N-(1-S-Carboxy-3-phenyl-propyl)-S-alanyl-1S,3S,5S-2-azabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

N-(1-S-Carboxy-3-phenyl-propyl)-S-alanyl-1S,3S,5S-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

Beispiel 8

Tabletten, die zur Behandlung der cardialen sowie der vasculären Hypertrophie und Hyperplasie verwendet werden können, werden wie in Beispiel 1 beschrieben hergestellt, mit der Ausnahme, daß anstelle von N-(1-S-Carbethoxy-3-phenyl-propyl)-S-alanyl-1S,3S,5S-2-azabicyclo[3.3.0]octan-3S-carbonsäure
N-(1-S-Carboxy-3-phenyl-propyl)-S-alanyl-1S,3S,5S-2-azabicyclo[3.3.0]octan-3-carbonsäure oder
N-(1-S-Carboxy-3-phenyl-propyl)-S-alanyl-2S,3aR,7aS-octahydroindol-2-carbonsäure oder
N-(1-S-Carbethoxy-3-phenyl-propyl)-S-alanyl-cis-2,3,3a,4,5,7a-hexahydro[1H]indol-2-S-endo-carbonsäure oder
N-(1-S-Carboxy-3-phenyl-propyl)-S-alanyl-cis-2,3,4a,4, 5,7a-hexahydro[1H]indol-2S-endo-carbonsäure oder
N-(1-S-Carboxy-3-phenyl-propyl)-S-lysyl-1S,3S,5S-2-azabicyclo[3.3.0]octan-3-carbonsäure oder
N-(1-S-Carbethoxy-3-cyclohexyl-propyl)-S-alanyl-1S,3S,5S-2-azabicyclo[3.3.0]octan-3-carbonsäure oder
N-(1-S-Carboxy-3-cyclohexyl-propyl)-S-lysyl-1S-3S,5S-2-azabicylco[3.3.0]octan-3-carbonsäure angewendet werden.

Beispiel 9

Eine Injektionslösung wird analog der in Beispiel 4 beschriebenen Vorschrift hergestellt, mit der Ausnahme, daß anstelle von N-(1-S-Carbethoxy-3-phenyl-propyl)-S-alanyl-1S,3S,5S-2-azabicyclo[3.3.0]octan-3-carbonsäure
N-(1-S-Carboxy-3-phenyl-propyl)-S-alanyl-1S,3S,5S-2-azabicyclo[3.3.0]octan-3-carbonsäure oder
N-(1-S-Carbethoxy-3-phenyl-propyl)-S-alanyl-2S,3aR,7aS-octahydroindol-2-carbonsäurehydrochlorid oder

N-(1-S-Carboxy-3-phenyl-propyl)-S-alanyl-2S,3aR,7aS-octahydroindol-2-carbonsäure oder
N-(1-S-Carbethoxy-3-cyclohexyl-propyl)-S-alanyl-cis-2,3,3a,4,5,7a-hexahydro[1H]indol-2-S-endo-carbonsäure oder
N-(1-S-Carboxy-3-phenyl-propyl)-S-alanyl-cis-2,3,3a,4,5,7a-hexahydro[1H]indol-2-S-endo-carbonsäure oder
N-(1-Carboxy-3-phenyl-propyl)-S-lysyl-1S,3S,5S-2-azabicyclo[3.3.0]octan-3-carbonsäure oder
N-(1-S-Carbethoxy-3-cyclohexyl)-S-alanyl-1S,3S,5S-2-azabicyclo[3.3.0]octan-3-carbonsäure oder
N-(1-S-Carboxy-3-cyclohexyl-propyl)-S-lysyl-1S,3S,5S-2-azabicyclo[3.3.0]octan-3-carbonsäure angewendet werden.

## Patentansprüche

1. Verwendung eines Angiotensin-Converting-Enzyme-Inhibitors oder dessen physiologisch verträglichen Salzes zur Herstellung eines Arzneimittels für die Behandlung der cardialen sowie der vasculären Hypertrophie und Hyperplasie in Säugern, dadurch gekennzeichnet, daß Angiotensin-Converting-Enzyme-Inhibitoren der Formel I

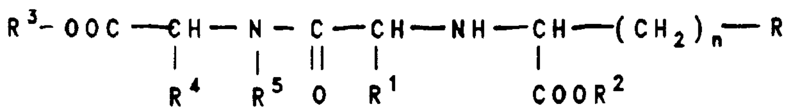

(I)

in welcher

n = 1 oder 2 ist,

R = Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen, das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben substituiert sein kann, ($C_6$-$C_{12}$)-Aryl-($C_1$-$C_4$)-alkylthio,
das im Arylteil wie oben bei Aryl beschrieben substituiert sein kann.
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,

$(C_6-C_{12})$-Aryloxy-$(C_1-C_4)$-alkyl,
das im Arylteil wie oben bei Aryl beschrieben substituiert sein kann oder $(C_6-C_{12})$-Aryl-$(C_1-C_4)$-alkoxy,
das im Arylteil wie oben bei Aryl beschrieben substituiert sein kann,

$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6-12 C-Atomen,
das wie oben bei R beschrieben substituiert sein kann,
$(C_6-C_{12})$-Aryl-$(C_1-C_4)$-alkyl oder $(C_7-C_{13})$-Aroyl-($C_1$ oder $C_2$)-alkyl, die beide wie das vorstehende Aryl substituiert sein können, mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder die gegebenenfalls geschützte Seitenkette einer natürlich vorkommenden $\alpha$-Aminosäure $R^1$-$CH(NH_2)$-COOH
bedeuten.

$R^2$ und $R^3$ gleich oder verschieden sind und Wasserstoff,
Alkyl mit 1-6 C-Atomen,
Alkenyl mit 2-6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy-$(C_1-C_4)$-alkyl,
Aryl mit 6-12 C-Atomen,
$(C_6-C_{12})$-Aryl-$(C_1-C_4)$-alkyl,
$(C_3-C_9)$-Cycloalkyl oder
$(C_3-C_9)$-Cycloalkyl-$(C_1-C_4)$-alkyl
bedeuten und worin

$R^4$ und $R^5$ zusammen mit den sie tragenden Atomen für ein gegebenenfalls substituiertes System aus der Reihe Tetrahydroisochinolin, Decahydroisochinolin, Octahydroindol, Octahydrocyclopenta(b)pyrrol, 2-Azaspiro(4,4)nonan, Spiro[(bicyclo(2.2.1)heptan)-2.3-pyrrolidin], Spiro[(bicyclo-(2,2,2)octan)-2.3-pyrrolidin], 2-Azatricyclo 4.3.0.$1^{6,9}$)decan,Decahydrocyclohepta(b)pyrrol, Octahydroisoindol, Octahydrocyclopenta(c)pyrrol, 2.3.3a.4.5.7a-Hexahydroindol und 2-Azabicyclo[3.1.0]hexan stehen, in einer sub-antihypertensiv wirksamen Dosierung eingesetzt werden.

2. Verwendung gemäß Anspruch 1, dadurch gekennzeichnet, daß (S.S.S.S.S)-N-(1-Carbethoxy-3-phenyl) propyl-alanyl-octahydroindol-2-carbonsäure angewendet wird.

3. Verwendung gemäß Anspruch 1, dadurch gekennzeichnet, daß N-[1-(S)-Carbethoxy-3-phenyl-propyl)-(S)-alanyl]-2S.3aR.7aS-octahydroindol-2-carbonsäure angewendet wird.

4. Verwendung gemäß Anspruch 1, dadurch gekennzeichnet, daß (S.S.S.S.S)-N-[(1-Carbethoxy-3-phenyl-propyl-alanyl]decahydroisochinolin-3-carbonsäure angewendet wird.

5. Verwendung gemäß Anspruch 1, dadurch gekennzeichnet, daß (S.S.S.S.S)-N-[(1-Carbethoxy-3-phenyl-propyl)-alanyl]tetrahydroisochinolin-3-carbonsäure angewendet wird.

6. Verwendung gemäß Anspruch 1, dadurch gekennzeichnet, daß (S.S.S.S.S)-N-(1-Carbethoxy-3-phenyl-propyl)-alanyl-2-azabicyclo(3.3.0)octan-3-carbonsäure angewendet wird.

7. Verwendung gemäß Anspruch 1, dadurch gekennzeichnet, daß N-(1-S-Carbethoxy-3-phenyl-propyl)-S-alanyl-cis-endo-2-azabicyclo(3. 1.0)hexan-3-S-carbonsäure angewendet wird.

8. Verwendung gemäß Anspruch 1, dadurch gekennzeichnet, daß N-(1-S-Carbethoxy-3-phenyl-propyl)-S-alanyl-cis-endo-2.3.3a.4.5.7a-hexahydroindol-2-S-carbonsäure angewendet wird.

9. Verwendung gemäß einem der Ansprüche 2 bis 8, dadurch gekennzeichnet, daß anstelle der Ethylester die entsprechenden Dicarbonsäuren angewendet werden.

10. Verwendung gemäß Anspruch 1, dadurch gekennzeichnet, daß die Angiotensin-Converting-Enzyme-Inhibitoren oral oder parenteral angewendet werden.

11. Verwendung gemäß Anspruch 1, dadurch gekennzeichnet, daß die Angiotensin-Converting-Enzyme-Inhibitoren für die entsprechenden Anwendungsformen mit pharmazeutisch geeigneten Trägerstoffen bzw Hilfsstoffen kombiniert werden.

## Claims

1. The use of an angiotensin converting enzyme inhibitor or physiologically tolerated salt thereof for the preparation of a medicament for the treatment of cardiac and of vascular hypertrophy and hyperplasia in mammals, wherein angiotensin converting enzyme inhibitors of the formula I

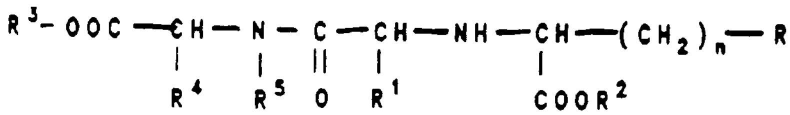

(I)

in which

n is 1 or 2,

R is hydrogen,

alkyl with 1-8 carbon atoms, alkenyl with 2-6 carbon atoms, cycloalkyl with 3-9 carbon atoms, aryl which has 6-12 carbon atoms and can be mono-, di- or trisubstituted by $(C_1-C_4)$-alkyl, $(C_1-C_4)$-alkoxy, hydroxyl, halogen, nitro, amino, aminomethyl, $(C_1-C_4)$-alkylamino, di-$(C_1-C_4)$-alkylamino, $(C_1-C_4)$-alkanoylamino, methylenedioxy, carboxyl, cyano and/or sulfamoyl, alkoxy with 1-4 carbon atoms,

aryloxy which has 6-12 carbon atoms and can be substituted as described above for aryl,

mono- or bicyclic heteroaryloxy which has 5-7 or 8-10 ring atoms, of which 1 to 2 ring atoms are sulfur or oxygen atoms and/or 1 to 4 ring atoms are nitrogen, and which can be substituted as described above for aryl,

amino-$(C_1-C_4)$-alkyl, $(C_1-C_4)$-alkanoylamino-$(C_1-C_4)$-alkyl, $(C_7-C_{13})$-aroylamino-$(C_1-C_4)$-alkyl, $(C_1-C_4)$-alkoxycarbonylamino-$(C_1-C_4)$-alkyl, $(C_6-C_{12})$-aryl-$(C_1-C_4)$-alkoxycarbonylamino-$(C_1-C_4)$alkyl, $(C_6-C_{12})$-aryl-$(C_1-C_4)$-alkylamino-$(C_1-C_4)$-alkyl, $(C_1-C_4)$-alkylamino-$(C_1-C_4)$-alkyl, di-$(C_1-C_4)$-alkylamino-$(C_1-C_4)$-alkyl, guanidino-$(C_1-C_4)$-alkyl, imidazolyl, indolyl, $(C_1-C_4)$-alkylthio,

$(C_1-C_4)$-alkylthio-$(C_1-C_4)$-alkyl,

$(C_6-C_{12})$-arylthio-$(C_1-C_4)$-alkyl which can be substituted in the aryl moiety as described above for aryl,

$(C_6-C_{12})$-aryl-$(C_1-C_4)$-alkylthio which can be substituted in the aryl moiety as described above for aryl,

carboxy-$(C_1-C_4)$-alkyl, carboxyl, carbamoyl, carbamoyl-$(C_1-C_4)$-alkyl, $(C_1-C_4)$-alkoxycarbonyl-$(C_1-C_4)$-alkyl,

$(C_6-C_{12})$-aryloxy-$(C_1-C_4)$-alkyl which can be substituted in the aryl moiety as described above for aryl, or

$(C_6-C_{12})$-aryl-$(C_1-C_4)$-alkoxy which can be substituted in the aryl moiety as described above for aryl,

$R^1$ is hydrogen, alkyl with 1-6 carbon atoms, alkenyl with 2-6 carbon atoms, alkynyl with 2-6 carbon atoms, cycloalkyl with 3-9 carbon atoms, cycloalkenyl with 5-9 carbon atoms, $(C_3-C_9)$-cycloalkyl-$(C_1-C_4)$-alkyl, $(C_5-C_9)$-cycloalkenyl-$(C_1-C_4)$-alkyl, optionally partially hydrogenated aryl which has 6-12 carbon atoms and can be substituted as described above for R,

$(C_6-C_{12})$-aryl-$(C_1-C_4)$-alkyl or $(C_7-C_{13})$-aroyl-($C_1$ or $C_2$)-alkyl, both of which can be substituted like the aryl above,

mono- or bicyclic, optionally partially hydrogenated heteroaryl which has 5-7 or 8-10 ring atoms, of which 1 to 2 ring atoms are sulfur or oxygen atoms and/or 1 to 4 ring atoms are nitrogen atoms, and which can be substituted like the aryl above, or the optionally protected side-chain of a naturally occurring $\alpha$-amino acid $R^1$-$CH(NH_2)$-COOH,

$R^2$ and $R^3$ are identical or different and are hydrogen, alkyl with 1-6 carbon atoms, alkenyl with 2-6 carbon atoms, di-($C_1$-$C_4$)-alkylamino-($C_1$-$C_4$)-alkyl, ($C_1$-$C_5$)-alkanoyloxy-($C_1$-$C_4$)-alkyl, ($C_1$-$C_6$)-alkoxycarbonyloxy-($C_1$-$C_4$)-alkyl, ($C_7$-$C_{13}$)-aroyloxy-($C_1$-$C_4$)-alkyl, ($C_6$-$C_{12}$)-aryloxycarbonyloxy-($C_1$-$C_4$)-alkyl,
aryl with 6-12 carbon atoms, ($C_6$-$C_{12}$)-aryl-($C_1$-$C_4$)-alkyl, ($C_3$-$C_9$)-cycloalkyl or ($C_3$-$C_9$)-cycloalkyl-($C_1$-$C_4$)-alkyl, and in which $R^4$ and $R^5$, together with the atoms carrying them, are an optionally substituted system from the series comprising tetrahydroisoquinoline, decahydroisoquinoline, octahydroindole, octahydrocyclopenta[b]pyrrole, 2-azaspiro(4,4) nonane, spiro[(bicyclo(2,2,1)heptane)2,3-pyrrolidine], spiro[(bicyclo(2,2,2)octane)-2.3'-pyrrolidine], 2-azatricyclo(4.3.0.$1^{6.9}$]decane, decahydrocyclohepta(b)pyrrole, octahydroisoindole, octahydrocyclopenta(c)pyrrole, 2.3.3a.4.5.7a-hexahydroindole and 2-azabicyclo[3.1.0]hexane,

are used in a sub-antihypertensively effective dose.

2. The use as claimed in claim 1, wherein (S.S.S.S.S)-N-(1-carbethoxy-3-phenyl)propyl-alanyl-octahydroindole-2-carboxylic acid is administered.

3. The use as claimed in claim 1, wherein N-(1-(S)-carbethoxy-3-phenyl-propyl-(S)-alanyl]-2S.3aR.7aS-octahydroindole-2-carboxylic acid is administered.

4. The use as claimed in claim 1, wherein (S.S.S.S.S)-N-[(1-carbethoxy-3-phenyl-propyl-alanyl]-decahydroisoquinoline-3-carboxylic acid is administered.

5. The use as claimed in claim 1, wherein (S.S.S.S.S)-N-[(1-carbethoxy-3-phenyl-propyl)-alanyl]-tetrahydroisoquinoline-3-carboxylic acid is administered.

6. The use as claimed in claim 1, wherein (S.S.S.S.S)-N-(1-carbethoxy-3-phenyl-propyl)-alanyl-2-azabicyclo-(3.3.0)octane-3-carboxylic acid is administered.

7. The use as claimed in claim 1, wherein N-(1-S-carbethoxy-3-phenyl-propyl)-S-alanyl-cis-endo-2-azabicyclo(3.1.0)hexane-3-S-carboxylic acid is administered.

8. The use as claimed in claim 1, wherein N-(1-S-carbethoxy-3-phenyl-propyl)-S-alanyl-cis-endo-2.3.3a.4.5.7a-hexahydroindole-2-S-carboxylic acid is administered.

9. The use as claimed in any of claims 2 to 8, wherein, in place of the ethyl esters, the corresponding dicarboxylic acids are administered.

10. The use as claimed in claim 1, wherein the angiotensin converting enzyme inhibitors are administered orally or parenterally.

11. The use as claimed in claim 1, wherein the angiotensin converting enzyme inhibitors are combined with pharmaceutically suitable vehicles and auxiliaries for the forms appropriate for administration.

## Revendications

1. Utilisation d'un inhibiteur de l'enzyme de conversion de l'angiotensine ou d'un de ses sels physiologiquement acceptables, pour la fabrication d'un médicament pour le traitement de l'hypertrophie et de l'hyperplasie cardiaques et vasculaires chez des mammifères, caractérisée en ce que l'on utilise à une dose inférieure à une dose à action antihypertensive des inhibiteurs de l'enzyme de conversion de l'angiotensine, de formule I

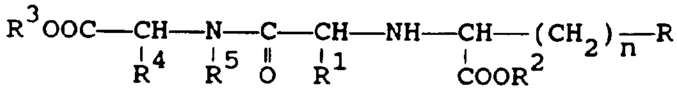 (I)

dans laquelle

n est 1 ou 2,

R représente un atome d'hydrogène,
un radical alkyle ayant de 1 à 8 atomes de carbone,
alcényle ayant de 2 à 6 atomes de carbone,
cycloalkyle ayant de 3 à 9 atomes de carbone,
aryle ayant de 6 à 12 atomes de carbone,
qui peut être mono-, di- ou trisubstitué par un ou des atomes d'halogène ou groupes alkyle en $C_1$-$C_4$, alcoxy en $C_1$-$C_4$, hydroxy, nitro, amino, aminométhyle, alkyl($C_1$-$C_4$)amino, dialkyl($C_1$-$C_4$)amino, alcanoyl($C_1$-$C_4$)amino,
méthylènedioxy, carboxy, cyano et/ou sulfamoyle,
un radical alcoxy ayant de 1 à 4 atomes de carbone,
un radical aryloxy ayant de 6 à 12 atomes de carbone,
qui peut être substitué comme décrit plus haut à propos du radical aryle,
un radical hétéroaryloxy mono- ou bicyclique ayant respectivement 5-7 ou 8-10 atomes formant le ou les cycles,
dont 1 ou 2 atomes formant le cycle représentent des atomes de soufre ou d'oxygène et/ou 1 à 4 atomes formant le cycle représentent des atomes d'azote, qui peut être substitué comme décrit plus haut à propos du radical aryle,
un radical amino-alkyle($C_1$-$C_4$),
alcanoyl($C_1$-$C_4$)amino-alkyle($C_1$-$C_4$),
aroyl($C_7$-$C_{13}$)amino-alkyle($C_1$-$C_4$),
alcoxy($C_1$-$C_4$)carbonylamino-alkyle($C_1$-$C_4$),
aryl($C_6$-$C_{12}$)-alcoxy($C_1$-$C_4$)carbonylamino-alkyle($C_1$-$C_4$),
aryl($C_6$-$C_{12}$)-alkyl($C_1$-$C_4$)amino-alkyle($C_1$-$C_4$),
alkyl($C_1$-$C_4$)amino-alkyle($C_1$-$C_4$),
dialkyl($C_1$-$C_4$)amino-alkyle($C_1$-$C_4$),
guanidino-alkyle($C_1$-$C_4$),
imidazolyle, indolyle,
alkyl($C_1$-$C_4$)thio,
alkyl($C_1$-$C_4$)thio-alkyle($C_1$-$C_4$),
un radical aryl($C_6$-$C_{12}$)thio-alkyle($C_1$-$C_4$) qui peut être substitué sur le fragment alkyle comme décrit plus haut à propos du radical aryle,
un radical aryl($C_6$-$C_{12}$)-alkyl($C_1$-$C_4$)thio qui peut être substitué sur le fragment aryle comme décrit plus haut à propos du radical aryle,
un radical carboxy-alkyle($C_1$-$C_4$),
carboxy, carbamoyle,
carbamoyl-alkyle($C_1$-$C_4$),
alcoxy($C_1$-$C_4$)carbonyl-alkyle($C_1$-$C_4$),
un radical aryloxy($C_6$-$C_{12}$)-alkyle($C_1$-$C_4$) qui peut être substitué sur le fragment aryle comme décrit plus haut à propos du radical aryle, ou
un radical aryl($C_6$-$C_{12}$)-alcoxy($C_1$-$C_4$) qui peut être substitué sur le fragment aryle comme décrit plus haut à propos du radical aryle,

$R^1$ représente un atome d'hydrogène,
un radical alkyle ayant de 1 à 6 atomes de carbone,
alcényle ayant de 2 à 6 atomes de carbone,
alcynyle ayant de 2 à 6 atomes de carbone,
cycloalkyle ayant de 3 à 9 atomes de carbone,
cycloalcényle ayant de 5 à 9 atomes de carbone,
cycloalkyl($C_3$-$C_9$)-alkyle($C_1$-$C_4$),
cycloalcényl($C_5$-$C_9$)-alkyle($C_1$-$C_4$),
un radical aryle ayant de 6 à 12 atomes de carbone, éventuellement partiellement hydrogéné, qui peut être substitué comme décrit plus haut à propos de R,
un radical aryl($C_6$-$C_{12}$)-alkyle($C_1$-$C_4$) ou aroyl($C_7$-$C_{13}$)-alkyle($C_1$ ou $C_2$) qui peuvent l'un et

l'autre être substitués comme le radical aryle précédent, un radical hétéroaryle mono- ou bicyclique, éventuellement partiellement hydrogéné, ayant respectivement 5-7 ou 8-10 atomes formant le ou les cycles, dont 1 ou 2 atomes formant le cycle représentent des atomes de soufre ou d'oxygène et/ou 1 à 4 atomes formant le cycle représentent des atomes d'azote, qui peut être substitué comme le radical aryle précédent, ou la chaîne latérale éventuellement protégée d'un $\alpha$-aminoacide $R^1$-CH($NH_2$)-COOH existant dans la nature,

$R^2$ et $R^3$ sont identiques ou différents et représentent un atome d'hydrogène,
un radical alkyle ayant de 1 à 6 atomes de carbone,
alcényle ayant de 2 à 6 atomes de carbone,
dialkyl($C_1$-$C_4$)amino-alkyle($C_1$-$C_4$),
alcanoyloxy($C_1$-$C_5$)-alkyle($C_1$-$C_4$),
alcoxy($C_1$-$C_6$)-carbonyloxy-alkyle($C_1$-$C_4$),
aroyloxy($C_7$-$C_{13}$)-alkyle($C_1$-$C_4$),
aryloxy($C_6$-$C_{12}$)-carbonyloxy-alkyle($C_1$-$C_4$),
aryle ayant de 6 à 12 atomes de carbone,
aryl($C_6$-$C_{12}$)-alkyle($C_1$-$C_4$),
cycloalkyle en $C_3$-$C_9$ ou
cycloalkyl($C_3$-$C_9$)-alkyle($C_1$-$C_4$),

et dans laquelle

$R^4$ et $R^5$ représentent, conjointement avec les atomes qui les portent, un système éventuellement substitué, choisi parmi les suivants: tétrahydroisoquinoléine, décahydroisoquinoléine, octahydroindole, octahydrocyclopenta(b)pyrrole, 2-azaspiro[4.4]nonane, spiro[(bicyclo[2.2.1]heptane)-2,3-pyrrolidine], spiro[(bicyclo[2.2.2]octane)-2,3-pyrrolidine], 2-azatricyclo(4.3.0.1$^{6,9}$]décane, décahydrocyclohepta(b)pyrrole, octahydroisoindole, octahydrocyclopenta(c)pyrrole, 2,3,3a,4,5,7a-hexahydroindole et 2-azabicyclo[3.1.0]hexane.

2. Utilisation selon la revendication 1, caractérisée en ce que l'on utilise l'acide (S,S,S,S,S)-N-(1-carbéthoxy-3-phényl)propylalanyl-octahydroindol-2-carboxylique.

3. Utilisation selon la revendication 1, caractérisée en ce que l'on utilise l'acide N-(1-(S)-carbéthoxy-3-phénylpropyl)-(S)-alanyl]-(2S,3aR,7aS)-octahydroindol-2-carboxylique.

4. Utilisation selon la revendication 1, caractérisée en ce que l'on utilise l'acide (S,S,S,S,S)-N-[(1-carbéthoxy-3-phénylpropyl)-alanyl]-décahydroisoquinoléine-3-carboxylique.

5. Utilisation selon la revendication 1, caractérisée en ce que l'on utilise l'acide (S,S,S,S,S)-N-[(1-carbéthoxy-3-phénylpropyl)-alanyl]-tétrahydroisoquinoléine-3-carboxylique.

6. Utilisation selon la revendication 1, caractérisée en ce que l'on utilise l'acide (S,S,S,S,S)-N-[(1-carbéthoxy-3-phénylpropyl)-alanyl-2-azabicyclo[3.3.0]octane3-carboxylique.

7. Utilisation selon la revendication 1, caractérisée en ce que l'on utilise l'acide N-(1-S-carbéthoxy-3-phénylpropyl)-S-alanyl-cis-endo-2-azabicyclo[3.1.0]hexane-3-S-carboxylique.

8. Utilisation selon la revendication 1, caractérisée en ce que l'on utilise l'acide N-(1-S-carbéthoxy-3-phénylpropyl)-S-alanyl-cis-endo-2,3,3a,4,5,7a-hexahydroindol-2-S-carboxylique.

9. Utilisation selon l'une des revendications 2 à 8, caractérisée en ce qu'au lieu des esters éthyliques, on utilise les acides dicarboxyliques correspondants.

10. Utilisation selon la revendication 1, caractérisée en ce que les inhibiteurs de l'enzyme de conversion de l'angiotensine sont utilisés par voie orale ou parentérale.

11. Utilisation selon la revendication 1, caractérisée en ce que, pour les formes d'administration correspondantes, on associe les inhibiteurs de l'enzyme de conversion de l'angiotensine avec des véhicules ou adjuvants pharmaceutiques appropriés.